# EUROPEAN PATENT SPECIFICATION

(11) **EP 1 765 251 B1**
(45) Date of publication and mention of the grant of the patent: **10.07.2013**
(21) Application number: 05754821.6
(22) Date of filing: 24.05.2005
(51) Int. Cl.: A61F 13/539

(54) **STABILIZED ABSORBENT STRUCTURES**
STABILISIERTE SAUGFÄHIGE STRUKTUREN
STRUCTURES ABSORBANTES STABILISEES

(30) Priority: 30.06.2004 US 883378
(43) Date of publication of application: 28.03.2007
(73) Proprietor: KIMBERLY-CLARK WORLDWIDE, INC., Neenah, WI 54956 (US)
(72) Inventor: SCHLINZ, Daniel, R., Greenville, Wisconsin 54952 (US); GOSSEN, Barbara, A., Oshkosh, Wisconsin 54904 (US); BLENKE, Timothy, J., Neenah, Wisconsin 54956 (US)
(74) Representative: Davies, Christopher Robert
(86) International application number: PCT/US2005/018309
(87) International publication number: WO 2006/007185

(56) References cited:
- EP-A- 0 829 245
- WO-A-01/45616

## Description

### BACKGROUND

The present invention relates to stabilized absorbent structures. More particularly, the present invention relates to stabilized absorbent structures having relatively high concentrations of superabsorbent material, the stabilized absorbent structures being suitable for incorporation into a variety of disposable absorbent articles.

Disposable care products such as diapers, children's training pants, adult incontinence pads and garments, menstrual pads, bed pads, surgical drapes and the like are typically comprised of at least three general layers. These include an absorbent core placed between a liquid permeable inner liner and a liquid impermeable outer cover. The inner liner and outer cover can comprise one or more individual layers of materials, and additional layers can also be interposed between any of the general layers. For example, in a disposable diaper, the inner liner can comprise a surge layer consisting of thermoplastic fibers positioned beneath a thermoplastic mesh. Additionally, a tissue material, or non-woven wrap sheet, is often positioned between the outer cover and absorbent core, and between the surge layer and the absorbent core. At the diaper periphery, the material layers extending to the periphery are held together by conventional means, such as adhesives, crimping, fusing, or other methods known in the art.

The absorbent core receives and retains bodily fluids. Traditional absorbent cores consist of a natural fiber batt that has a strong affinity for water and other hydrophilic components of bodily secretions. A dispersion of superabsorbent particles can also be incorporated into the fibrous absorbent core.

Many traditional absorbent cores that include a high-absorbency material includes the superabsorbent particles in relatively low concentrations. That is, many of the absorbent cores include airlaid cellulosic fibers and less than about 50 weight percent of a superabsorbent material.

However, in recent years it has become increasingly desirable to produce absorbent cores which are thin compared to the more traditional absorbent composites but which still possess the same absorbent capacity. Thin absorbent cores provide for a greater garment-like appearance as well as improved discretion when worn under other garments. The desire to produce relatively thin absorbent composites has resulted in the desire to incorporate ever-increasing amounts of superabsorbent material into the absorbent cores. This is because the absorbent capacity of such superabsorbent materials is generally many times greater than the absorbent capacity of fibrous materials. For example, a fibrous matrix of wood pulp fluff can absorb about 7-9 grams of a liquid per gram of wood pulp fluff, while the superabsorbent materials can absorb at least about 15, preferably at least about 20, and often at least about 25 grams of liquid per gram of the superabsorbent material.

As the superabsorbent material is contacted with fluid, it can swell to a significant volume. This can cause problems with absorbent cores, especially those containing high concentrations of superabsorbent materials.

Maintaining a continuous, intact absorbent core, especially when these articles contain high percentages of superabsorbent material, is a recurring issue in the disposable garment industry. Breaks in the continuum of the absorbent core create open spaces that prevent the transport of fluid into the core, and the wicking of the fluid in the core. This breakdown of the absorbent core structure can cause fluids to leak out of the periphery of the diaper. The absorbent core breakdown also results in its sagging, which is visually unappealing to the consumer. The flowability of the superabsorbent material further contributes to the sagging of the absorbent core. Migration of the superabsorbent material either before or after saturation may result in the sagging or breakdown of the absorbent core.

Prior absorbent cores have involved the formation of discrete, spaced pockets of superabsorbent material. See U.S. Patent 4,360,021 issued on November 23, 1982 to Stima (backsheet 13 and cover sheet 15 made of tissue wadding that is selectively compressed to form pockets of hydrogel- forming absorbent polymer particles 24), U.S. Patent 4,994,053 issued on February 19, 1991 to Lang (pattern of adhesive 60, as well as heat fusible films 68 and 70, to form pockets of hydrogel-forming absorbent polymer particles 56/58), U.S. Patent 4,055,180 issued on October 25, 1977 to Karami (retaining sheet 56 that is preferably a thermoplastic film fused to wadding sheet 40 by heat to form pockets of hydrogel-forming absorbent polymer particles 62), and U.S. Patent 5,149,335 issued on September 22, 1992 to Kellenberger et al. (outer cover 22 bonded to body-side liner 24 along bond lines 28 to form compartments 30 of hydrogel-forming absorbent polymer particles 32).

Absorbent cores having these discrete pockets of particles form an essentially "discontinuous" layer or pattern of superabsorbent material. Since the superabsorbent material is a discontinuous layer, it may be more difficult for the fluid to reach each of the discrete pockets. Indeed, in some of these "discontinuous" laminate structures, the discrete pockets of superabsorbent material are not in direct fluid communication. Moreover, these discrete pockets of superabsorbent material can form protuberances that are undesirable aesthetically and can make the absorbent article uncomfortable for the wearer.

Some prior laminate structures have involved continuous regions of superabsorbent material that surround multiple, spaced apart zones which are substantially devoid of superabsorbent material. These structures are formed by superimposing a first layer on a porous screen having multiple spaced apart, nonporous, protruding areas corresponding to the areas desired for the zones that are to be substantially devoid of superabsorbent material. A vacuum is introduced under the porous screen opposite the superimposed layer, and parties of superabsorbent material are substantially uniformly distributed across the length and width of the superimposed layer. The particles are then caused by the combination of vacuum and protruding areas to migrate to the areas between the protruding areas to form a substantially uniform distribution in a continuous region on the layer which surrounds multiple zones which are substantially devoid of superabsorbent material. Thereafter, the particles are misted with a water spray, and a second layer is juxtaposed in facing relation onto the first layer to cover the superabsorbent material. Finally, the two layers are bonded together at locations within a plurality of the zones that are substantially devoid of superabsorbent material.

EP-0829245 discloses a bonded absorbent core structure. WO 01/45616 discloses a laminate structure comprising a material disposed between first and second non wovens which are joined at a plurality of discrete bond sites.

Accordingly, it would be desirable to provide absorbent structures that: have highs concentrations of superabsorbent material; can immobilize the superabsorbent material without restricting its ability to expand as it is contacted with additional fluid; and are relatively easy to manufacture.

The present inventors undertook intensive research and development efforts concerning absorbent structures and stabilized absorbent structures. While conducting their research, the present inventors discovered unique absorbent structures adapted to contains aqueous body exudates such as urine, menses and loose bowel movements. According to the present invention there is provided an absorbent structure as defined in claim 1.

One versions of the present invention involves an absorbent structure having opposing first and second layers, the first layer comprising thermoplastic material, the second layer comprising thermoplastic material. The absorbent structure also has an absorbent core located between the first and second layers. Further, the first layer and the second layer of the absorbent structure have thermoplastic point bonds therein, wherein at least a portion of the thermoplastic point bonds are liquid stable, and at least a portion of the thermoplastic point bonds operatively join the first and second layers through the absorbent core.

Another version of the present invention relates to a disposable absorbent article having an outer cover, a liner superposed over at least a portion of the outer cover; and an absorbent structure. The absorbent structure having opposing first and second layers, the first layer having thermoplastic material, the second layer having thermoplastic material, and an absorbent core located between the first and second layers. Further, the first layer and the second layer of the absorbent structure having thermoplastic point bonds therein, wherein at least a portion of the thermoplastic point bonds are liquid stable, and at least a portion of the thermoplastic point bonds operatively join the first and second layers through the absorbent core.

It is to be understood that both the foregoing general description and the following detailed description are exemplary and are intended to provide further explanation of the invention claimed. The accompanying drawings, that are incorporated in and constitute part of this specification, are included to illustrate and provide a further understanding of the articles of the invention. Together with the description, the drawings serve to explain various aspects of the invention.

### Drawings

The foregoing and other features, aspects and advantages of the present invention will become better understood with regard to the following description, appended claims and accompanying drawings where:
FIG. 1 illustrates a partially cut away, top plan view of an absorbent structure;
FIG. 2A illustrates a cross sectional view of an absorbent structure before point bonding;
FIG. 2B illustrates a cross sectional view of the absorbent structure of FIG 2A after point bonding;
FIGs. 3 and 4 illustrate cross sectional views of several versions of an absorbent structure;
FIGs. 5A, 5B and 5C illustrate cross sections views an absorbent structure in three stages of folding;
FIGs. 6 through 8 illustrate cross sectional views of several versions of an absorbent core including additional layers;
FIGs. 9 through 11 illustrate cross sectional views of several versions of disposable absorbent articles;
FIG. 12 representatively illustrates a partially cut-away, top plan view of the bodyfacing or upper surface of a disposable absorbent article incorporating a version of the absorbent structure.

### Detailed Description of the Invention

The present invention relates to absorbent structures (50), illustrative embodiments of which are shown in the Figures. The absorbent structures are adapted to contain aqueous body exudates such as urine, menses and loose bowel movements. In particular, the absorbent structures of the present invention are suitable for incorporation into a variety of disposable absorbent articles.

The present disclosure of the invention will be expressed in terms of its various components, elements, constructions, configurations, arrangements and other features that may also be individually or collectively be referenced by the term, "aspect(s)" of the invention, or other similar terms. It is contemplated that the various forms of the disclosed invention may incorporate one or more of its various features and aspects, and that such features and aspects may be employed in any desired, operative combination thereof.

It should also be noted that, when employed in the present disclosure, the terms "comprises", "comprising" and other derivatives from the root term "comprise" are intended to be open-ended terms that specify the presence of any stated features, elements, integers, steps, or components, and are not intended to preclude the presence or addition of one or more other features, elements, integers, steps, components, or groups thereof.

By "particle," "particles," "particulate," "particulates" and the like, it is meant that a material is generally in the form of discrete units. The particles can includes granules, pulverulents, powders or spheres. Thus, the particles can have any desired shape such as, for example, cubic, rod-like, polyhedral, spherical or semispherical, rounded or semi-rounded, angular, irregular, etc. Shapes having a large greatest dimension/smallest dimension ratio, like needles, flakes and fibers are also contemplated for use herein. The use of "particle" or "particulate" may also describe an agglomeration including more than one particle, particulate or the like. All percentages, ratios and proportions used herein are by weight unless otherwise specified.

In the various versions of the present invention, many suitable types of wettable, hydrophilic fibrous material can be used to form many of the various component parts of the absorbent structure (50). Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made from inherently wettable thermoplastic polymers, such as particular polyester or polyamide fibers; and synthetic fibers composed of a nonwettable thermoplastic polymer, such as polypropylene fibers. The fibers may be hydrophilized, for example, by treatment with silica, treatment with a material which has a suitable hydrophilic moiety and is not readily removable from the fiber, or by sheathing the nonwettable, hydrophobic fiber with a hydrophilic polymer during or after the formation of the fiber. For the purposes of the present invention, it is contemplated that selected blends of the various types of fibers mentioned above may also be employed.

As used herein, the term "hydrophilic" is intended to describe fibers or the surfaces of fibers which are wetted by the aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with such a system, fibers having contact angles less than 90° are designated "wettable", while fibers having contact angles equal to or greater than 90° are designated "nonwettable".

In particular, the absorbent structure (50) can include one or more matrices of fibers. Desirably the fibers are hydrophilic, either naturally or through the effects of a conventional hydrophilic treatment. Particular arrangements according to the present invention include a fibrous matrix composed of cellulosic woodpulp fluff.

According to the present invention, the fibers of the absorbent structure (50) are mixed or otherwise incorporated with particles of superabsorbent material. The fibers in the absorbent structure typically are arranged in an absorbent matrix combined with particles of a superabsorbent material. In certain arrangements, for example, the absorbent structure includes a mixture of particles of a superabsorbent material, natural fibers and synthetic polymer meltblown fibers (a fibrous coform material including a blend of natural fibers and/or synthetic polymer fibers). The superabsorbent material may be substantially homogeneously mixed with the hydrophilic fibers, or may be nonuniformly mixed. For example, the concentrations of the superabsorbent material may be arranged in a non-step-wise gradient through a substantial portion of the thickness (z-direction) of the absorbent structure, with lower concentrations toward the bodyside of the absorbent structure and relatively higher concentrations toward the outerside of the absorbent structure. Suitable z-gradient configurations are described in U.S. Patent No. 4,699,823 to Kellenberger et al. Alternatively, the concentrations of superabsorbent material may be arranged in a non-step-wise gradient, through a substantial portion of the thickness (z-direction) of the absorbent structure (50), with higher concentrations toward the bodyside of the absorbent structure (50) and relatively lower concentrations toward the outerside of the absorbent structure (50). The superabsorbent material may also be arranged in a generally discrete layer within the matrix of hydrophilic fibers. In addition, two or more different types of superabsorbent material may be selectively positioned art different locations within or along the fiber matrix.

A wide variety of materials can be suitably employed as the superabsorbent material of the present invention. It is desired, however, to employ superabsorbent materials in particle form capable of absorbing large quantities of fluids, such as water, and of retaining such absorbed fluids under moderate pressures.

As used herein, "superabsorbent material," "superabsorbent materials" and the like are intended to refer to a water-swellable, water-insoluble organic or inorganic material capable, under the most favorable conditions, of absorbing at least about 10 times its weight and, preferably, at least about 15 times its weight in an aqueous solution containing 0.9 weight percent of sodium chloride. Such materials include, but are not limited to, hydrogel-forming polymers which are alkali metal salts of: poly(acrylic acid); poly(methacrylic acid); copolymers of acrylic and methacrylic acid with acrylamide, vinyl alcohol, acrylic esters, vinyl pyrrolidone, vinyl sulfonic acids, vinyl acetate, vinyl morpholinone and vinyl ethers; hydrolyzed acrylonitrile grafted starch; acrylic acid grafted starch; maleic anhydride copolymers with ethylene, isobutylene, styrene, and vinyl ethers; polysaccharides such as carboxymethyl starch, carboxymethyl cellulose, methyl cellulose, and hydroxypropyl cellulose; poly(acrylamides); poly(vinyl pyrrolidone); poly(vinyl morpholinone); poly(vinyl pyridine); and copolymers and mixtures of any of the above and the like. The hydrogel-forming polymers are preferably lightly crosslinked to render them substantially water-insoluble. Cross-linking may be achieved by irradiation or by covalent, ionic, van der Waals attractions, or hydrogen bonding interactions, for example. A desirable superabsorbent material is a lightly crosslinked hydrocolloid. Specifically, a more desirable superabsorbent material is a partially neutralized polyacrylate salt. Processes for preparing synthetic, absorbent gelling polymers are disclosed in U.S. Patent No. 4,076,663, issued to Masuda et al., and U.S. Patent No. 4,286,082, issued to Tsubakimoto et al.

Superabsorbent materials employed in the present invention suitably should be able to absorb a liquid under an applied load. For purposes of this application, the ability of a superabsorbent material to absorb a liquid under an applied load and thereby perform work is quantified as the Absorbency Under Load (AUL) value. The AUL value is expressed as the amount (in grams) of an approximately 0.9 weight percent saline (sodium chloride) solution absorbed by about 0.160 grams of superabsorbent material when the superabsorbent material is under a load. Common loads, further described herein below, include those of about 0.29 pound per square inch, 0.57 pound per square inch, and about 0.90 pound per square inch. Superabsorbent materials suitable for use herein desirably are stiff-gelling superabsorbent materials having an AUL value under a load of about 0.29 pound per square inch of at least about 7; alternatively, at least about 9; alternatively, at least about 15; alternatively, at least about 20; alternatively, at least about 24; and, finally, alternatively, at least about 27 g/g. (Although known to those skilled in the art, the gel stiffness or shear modulus of a superabsorbent material is further described in U.S. Patent No. 5,147,343, issued to Kellenberger, and European Patent Office Publication No. 0339461, published November 2,1989). Useful superabsorbent materials are typically available from various commercial vendors, such as, for example, Dow Chemical Company or Stockhausen, Inc.

Suitably, the superabsorbent material is in the form of particles which, in the unswollen state, have maximum cross-sectional diameters ranging between about 50 and about 1,000 microns; desirably, between about 100 and about 800 microns; more desirably, between about 200 and about 650 microns; and most desirably, between about 300 and about 600 microns, as determined by sieve analysis according to American Society for Testing Materials Test Method D-1921. It is understood that the particles of superabsorbent material may include solid particles, porous particles, or may be agglomerated particles including many smaller particles agglomerated into particles falling within the described size range.

AUL values may be determined by a number of methods known to those of skill in the art. One such method is described in U.S. Patent No. 5,601,542, issued to Melius et al. AUL is believed to be a function of the following factors: (1) gel stiffness while swelling, (2) ability to imbibe the fluid by osmotic and internal electrostatic repulsion forces, (3) surface wettability of the superabsorbent material and (4) particle size distribution when wet.

Those of skill in the art will readily appreciate that other suitable arrangements for the absorbent structures (50) of the present invention include configurations having one or more layers. Each layer may include particles of superabsorbent material, non-superabsorbent material and/or combinations thereof. The layers of any such multi-layered absorbent structure (50) may be connected or otherwise associated together in an operable manner. As used herein when describing layers of an absorbent structure (50), the term "associated" is intended to encompass configurations in which two or more layers are directly in liquid communication with each other, as well as configurations where two or more layers are indirectly in liquid communication with each other by affixing portions of a layer to intermediate members or elements which in turn are affixed to at least portions of another layer. In addition to hydrophilic fibers, one or more layers of an absorbent structure (50) may include other non-superabsorbent material such as, for example, scrim material.

Suitably, the absorbent structures (50) of the present invention have a basis weight of no less than about 200; alternatively, no less than about 300, or finally, alternatively, no less than about 500 grams per square meter (gsm). Furthermore, the absorbent structures (50) of the present invention suitably have a basis weight of no greater than about 800; alternatively, no greater than about 700; or finally, alternatively, no greater than about 600 grams per square meter. Thus, suitable configurations of the absorbent structure (50) of the present invention may have basis weights ranging between no less than about 200 up to no greater than about 800 gsm; although the approximate basis weight may vary according to, *inter alia,* the general design and intended use of the absorbent structure (50).

The superabsorbent material is typically present in the absorbent structure (50) in an amount of no less than about 40; alternatively, no less than about 60; or finally, alternatively, no less than about 80 % by weight. In addition, the superabsorbent material is typically present in the absorbent structure (50) in an amount of no greater than about 99; alternatively, no greater than about 80; or finally, alternatively, no greater than about 60 % by weight. Thus, suitable configurations of the absorbent structure (50) of the present invention may have superabsorbent material present in an amount ranging between no less than about 40 up to no greater than about 99 % by weight; although the approximate amount of superabsorbent material may vary according to, *inter alia,* the general design and intended use of the absorbent structure (50).

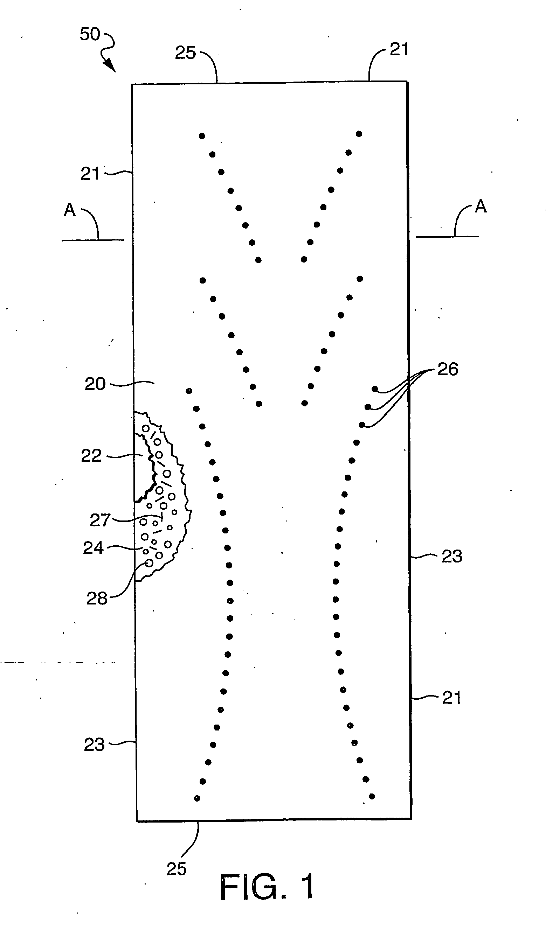

FIG. 1 illustrates an absorbent structure (50) as having a first layer (20) and an opposing second layer (22), and an absorbent core (24) located between the first layer (20) and the second layer (22). The first layer (20) comprises one major facing surface of the absorbent structure (50) and the second layer (22) comprises the second major facing surface of the absorbent structure. The absorbent structure (50) includes a plurality of point bonds (26) which form patterns on both the first (20) and second layers (22). The outer edges of the absorbent structure (50) define a periphery (21) with laterally opposed, longitudinally extending side edges (23); longitudinally opposed, laterally extending end edges (25). The absorbent core (24) includes a matrix of hydrophilic fibers (27), such as a web of cellulosic fluff, mixed with particles of a high-absorbency material commonly known as superabsorbent material (28). The point bonds (26) may be adhesive bonds, sonic bonds, thermal bonds, pressure bonds, pinning, stitching, or a variety of other attachment techniques known in the art, as well as combinations thereof. Desirably, the materials that form the point bond (26) form the bond autogenously (without additional bonding material such as adhesive).

The point bonds (26) are typically no less than about 1; alternatively, no less than about 2; or finally, alternatively, no less than about 3 mm in one dimension. In addition, the point bonds (26) are typically no greater than about 4; alternatively, no greater than about 3; or finally, alternatively, no greater than about 2 mm in one dimension. Thus, suitable configurations of the absorbent structure (50) of the present invention may have the point bonds (26) having a length in one dimension ranging between no less than about 1 up to no greater than about 4 mm; although the approximate dimension may vary according to, *inter alia,* the general design and intended use of the absorbent structure (50).

The point bonds (26) are typically no less than about 3; alternatively, no less than about 6; or finally, alternatively, no less than about 9 mm apart. In addition, the point bonds (26) are typically no greater than about 16; alternatively, no greater than about 12; or finally, alternatively, no greater than about 9 mm apart. Thus, suitable configurations of the absorbent structure (50) of the present invention may have the point bonds (26) having a spacing ranging between no less than about 3 up to no more than about 16 mm; although the approximate spacing may vary according to, *inter alia,* the general design and intended use of the absorbent structure (50).

In a particular version, the absorbent core (24) includes a mixture of superabsorbent hydrogel-forming particles and wood pulp fluff. The superabsorbent particles may be substantially homogeneously mixed with the hydrophilic fibers or may be non-uniformly mixed. One suitable type of wood pulp fluff is identified with the trade designation CR-1654, available from Bowater, Inc., Greenville, South Carolina, and is a bleached, highly absorbent sulfate wood pulp containing primarily soft wood fibers. A special densification pulp, identified with the trade designation ND-416, available from Weyerhaeuser of Federal Way, Washington, is also suitable for use.

The first layer (20) and the second layer (22) may be generally coextensive (e.g., FIG. 1), or optionally, may be non-coextensive. Either or both of the first layer (20) and the second layer (22) may have length and width dimensions which are generally larger than those of the absorbent core (24) and extend beyond the corresponding dimensions of the absorbent core (24) to provide longitudinal side edges (23) and lateral end edges (25) which may be connected or otherwise associated together in an operable manner. As used herein when describing the first layer (20) in relation to the second layer (22) and vice versa, the term "associated" encompasses configurations in which the first layer (20) is directly joined to the second layer (22), and configurations where the first layer (20) is indirectly joined to the second layer (22) by affixing portions of the first layer (20) to intermediate members which in turn are affixed to at least portions of the second layer (22). The first layer (20) and the second layer (22) can, for example, be joined to each other in at least a portion of the absorbent core periphery (21) by attachment mechanisms (not shown) such as adhesive bonds, sonic bonds, thermal bonds, pinning, stitching, or a variety of other attachment techniques known in the art, as well as combinations thereof. Alternatively, the absorbent core (24) may be wrapped by a single web, whereby, the first layer (20) and the second layer (22) are portions of the same web.

Various woven and nonwoven fabrics may comprise the first layer (20) and second layer (22). For example, the first layer (20) or second layer (22) may be composed of a meltblown or spunbonded web of polyolefin fibers. The first layer (20) or second layer (22) may also be a bonded-carded web composed of natural and/or synthetic fibers. The first layer (20) or second layer (22) may be composed of a substantially hydrophobic material, and the hydrophobic material may, optionally, be treated with a surfactant, or otherwise processed, to impart a desired level of wettability and hydrophilicity. Specifically, the first layer (20) or second layer (22) may be a nonwoven, spunbond-meltblown-spunbond, polypropylene fabric having a basis weight of about 5 to 30 gsm.

The first layer (20) and/or the second layer (22) may be stretchable, either elastically or extensible thereby allowing the absorbent core (24) to swell. In desired configurations, the first layer (20) or the second layer (22) can provide a stretch elongation as measured at a load of 250 g/in. Desirably the first layer (20) or the second layer (22) can provide a stretch elongation that is no less than about 20%, alternatively, no less than about 60%; and finally, alternatively, no less than about 100%. Desirably the first layer (20) or the second layer (22) can provide a stretch elongation measure at 250 g/in that are no more than about 120%; alternatively, no more than about 100%; and-finally, alternatively no more than about 50%. Thus, the first layer (20) or the second layer (22) typically can provide a stretch elongation measured at 250 g/in that is no less than about 20% and no more than about 120%; although the approximate percent may vary according to, *inter alia,* the general design and intended use of the first layer (20) or the second layer (22). Further, the stretch elongation of the first layer (20) may be different than the stretch elongation of the second layer (22).

The first layer (20) and second layer (22) may suitably be composed of a material which is either liquid permeable or liquid impermeable. It is generally desirable that at least one of the first layer (20) or second layer (22) be formed from a material which is substantially liquid permeable. The liquid permeability may be inherent in the web forming the layer (20, 22), in the example of a low basis weight spunbond, alternatively, the permeability may result from a web that is inherently liquid impermeable, for example a film, which has been modified to provide the permeability, for example by aperturing.

The first layer (20) and the second layer (22) may be provided by a similar material. Alternatively the first layer (20) and the second layer (22) may be provided by dissimilar materials. The first layer (20) may be adapted to face a user's body during use, and the second layer (22) may be adapted to face away from the use's body during use, if the absorbent structure (50) is utilized in a disposable absorbent article.

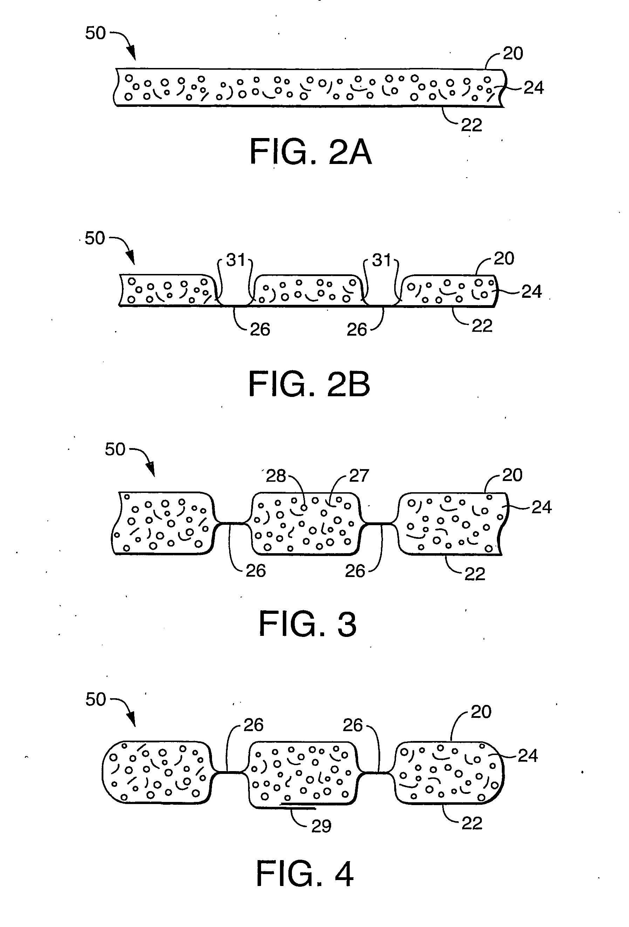

FIG. 2A illustrates a cross sectional view of an absorbent structure (50) before point bonding. FIG. 2B illustrates a cross sectional view of the absorbent structure of FIG 2A after point bonding. FIG. 2B also illustrates a cross sectional view of the absorbent structure (50) of FIG 1 as viewed from the line labeled A - A. FIG. 2A illustrates an absorbent structure (50) including a first layer (20), and a second layer (22) and an absorbent core (24) located between the first layer (20) and the second layer (22). The illustrated absorbent core (24) has a basis weight that is substantially uniform across the cross section.

FIG. 2B illustrates the absorbent core (24) of FIG. 2A after point bonds (26) have joined the first layer (20) and the second layer (22) through the absorbent core (24) with a point bond. The point bond may be liquid stable, alternatively the bond may not be liquid stable. As used herein, a bond this is "through the absorbent core" is a bond that is formed when a portion of the first layer (20) and a portion of the second layer (22) are brought into close proximity. Further, where the first and second layers (20, 22) are brought in close proximity, the absorbent core (24) is displaced into the margins (31) surrounding the bond. In particular embodiments where the absorbent core (24) contains superabsorbent material, the margins (31) surrounding the bond may contain pulverized superabsorbent material. In other embodiments, the absorbent core (24) in the margins (31) surrounding the bond may be compressed such that the density is greater than the density of the absorbent core (24) in areas other than in the margins (31) surrounding the bond.

As used herein, a bond that is "liquid stable" is a bond that remains intact when exposed to liquid, such as urine, water, menses, blood, or fecal material. Specifically excluded, are bonds which rely exclusively on hydrogen bonding for their strength. The point bonds (26) may contain sufficient "wet" strength such that when the absorbent structure (50) is completely saturated and swollen (with the fluid the absorbent structure is designed to contain) the point bonds (26) remain intact.

FIG. 2B illustrates a location of the point bond (26) in a z-direction of the absorbent structure (50). Specifically, FIG. 2B illustrates the point bond (26) located in the z-direction that corresponds with one of the surfaces of the absorbent structure (50), specifically the second layer (22). FIG. 3 illustrates a location the point bond (26) in a z-direction of the absorbent structure (50). Specifically, FIG. 3 illustrates the point bond (26) located in the z-direction that corresponds generally with a location centered between the first and second layers (20, 22). One skilled in the art will appreciate that the location of the point bond (26) in the z-direction of the absorbent structure (50) can be modified to provide a variety of functional and aesthetic results.

F1G. 4 illustrates a cross sectional view of an absorbent structure (50) wherein the first layer (20) and the second layer (22) are portions of a single web. The single web is wrapped around the absorbent core (24), and then overlapped forming a seam (29). As shown the seam (29) is centrally located on the second layer (22), however, the seam (29) may be located on the first layer (20) or the seam (29) may be located on the edge of the absorbent structure (50).

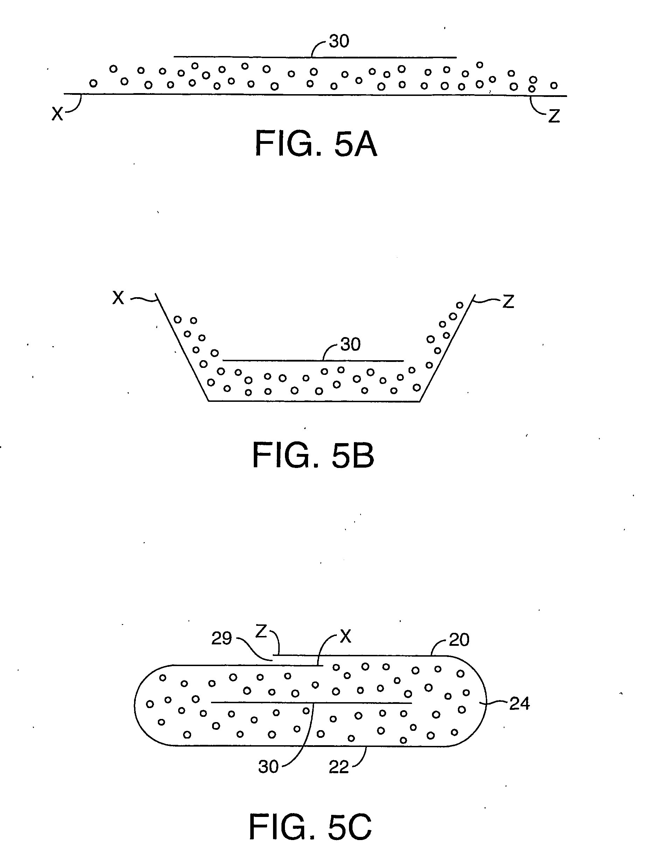

FIGs. 5A, 5B and 5C illustrate cross section views of an absorbent structure in three stages of folding. FIG. 5A illustrates a web of material with side edges (X, Z) on which a batt of absorbent material has been placed. Further, an additional layer (30) has been place on the center portion of the absorbent material. FIGS. 5B and 5C illustrate how the side edges (X, Z) are brought around the additional layer (30), and the edges (X, Z) are overlapped to form a seam (29). In this way, an additional layer (30) is located between the first layer (20) and second layer (22) with absorbent material located on top of and below the additional layer (30). This additional layer (30) may be a distribution layer, a surge layer, an intake layer, a scrim layer or other suitable layer. The material which the additional layer (30) is comprised may contribute to the point bond (26) when formed, and may strengthen the point bond (26). The additional layer (30) may be coextensive with the first or second layer (20, 22), alternatively, the additional layer (30) may be larger or smaller than the first or second layer (20, 22).

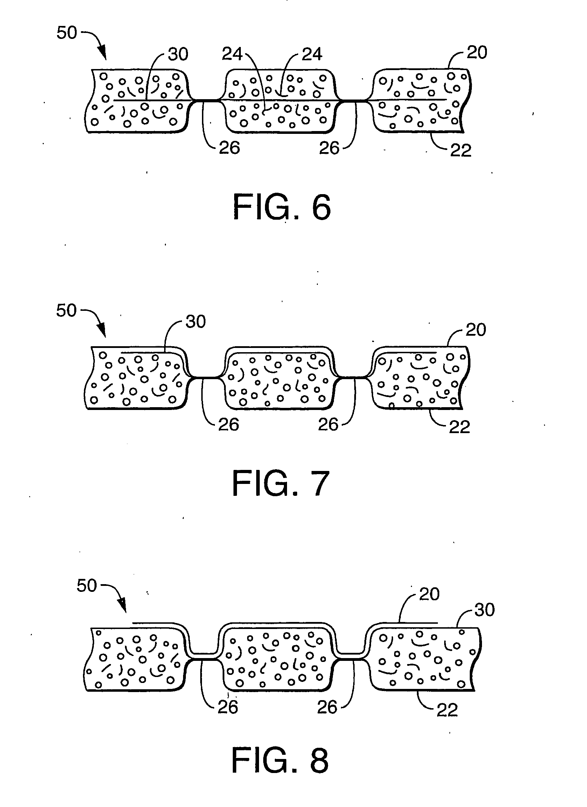

FIG. 6 illustrates a cross sectional view of an absorbent structure (50) wherein an additional layer (30) is located between the first layer (20) and the second layer (22) and portions of the absorbent core (24) are located between the additional layer (30) and the first layer (20) and the additional layer (30) and the second layer (22). FIG. 7 illustrates a cross sectional view of an absorbent structure (50) wherein an additional layer (30) is located between the first layer (20) and the second layer (22), and the additional layer (30) is located adjacent to the first layer (20). As shown in FIG. 7, the additional layer (30) is smaller than the first layer (20).

FIG. 8 illustrates a cross sectional view of an absorbent structure (50) wherein an additional layer (30) is located between the first layer (20) and the second layer (22), and the additional layer (30) is located adjacent to the first layer (20). As shown in FIG. 8, the additional layer (30) is lager than the first layer (20).

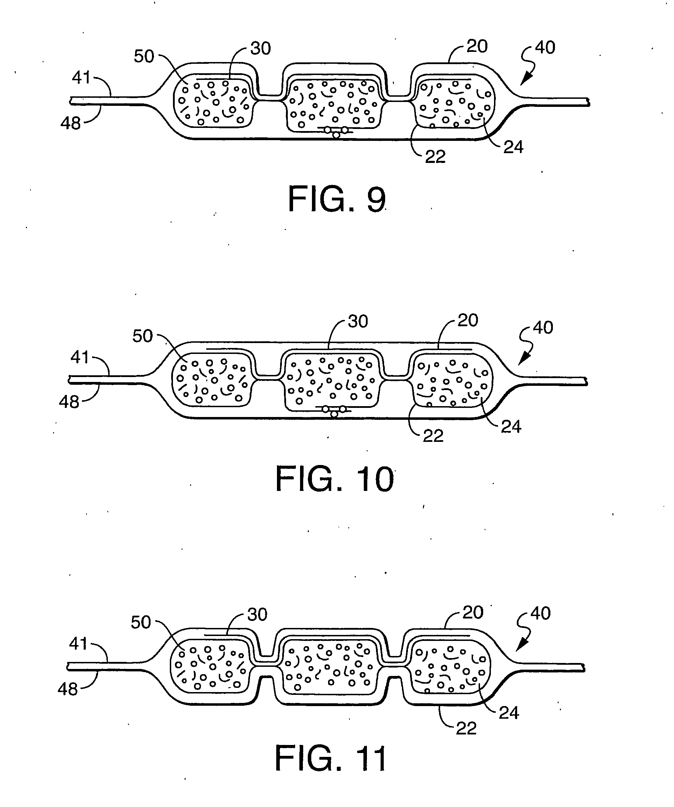

FIGs. 9 through 11 illustrate cross sectional views of several versions of disposable absorbent articles, specifically disposable diapers (40). The diapers (40) as illustrated in FIGs. 9 through 11 include a bodyside liner (41), an outer cover (48) and an absorbent structure (50) (which may or may not include the liner and/or outer cover). FIG. 9 illustrates a diaper (40) wherein the bodyside liner (41) is the first layer (20) of the absorbent structure (50). The absorbent structure (50) includes the bodyside liner (41), an absorbent core (24), first and second layers (20, 22) and an additional layer (30). The absorbent structure (50) may be attached to the outer cover (48) by means of an adhesive bead as shown, alternatively, the absorbent structure (50) may not be attached to the over cover (48), allowing the outer cover (48) to blouse away from the absorbent structure (50).

FIG. 10 illustrates a diaper (40) with a bodyside liner (41), an outer cover (48) and an absorbent structure (50) located between the bodyside liner (41) and the outer cover (48) which does not fall within the scope of the present invention. The absorbent structure (50) may be attached to the outer cover (48) for example with an adhesive bead as shown, alternatively, the absorbent structure (50) may not be attached to the over cover (48), allowing the outer cover (48) to blouse away from the absorbent structure (50). The bodyside liner (41) may be allowed to blouse away from the absorbent structure (50), as shown, or alternatively, the bodyside liner (41) may be attached to the absorbent structure (50) by any means known in the art.

FIG. 11 illustrates a diaper (40) wherein the bodyside liner (41) is the first layer (20) of the absorbent structure (50) and the outer cover (48) is the second layer (22) of the absorbent structure (50). The absorbent structure (50) includes the bodyside liner (41), an absorbent core (24), first and second layers (20, 22), an additional layer (30) and the outer cover (48).

The various aspects, benefits, and versions of the absorbent structure (50) have been described in the context of a disposable absorbent article, such as a disposable diaper. It is, however, readily apparent that one or more versions of the present invention could also be employed with other disposable absorbent articles, such as feminine hygiene articles, children's training pants, adult incontinence garments, bed pads, surgical drapes and the like. Typically, disposable absorbent articles are intended for limited use and are not intended to be laundered or otherwise cleaned for reuse. A disposable diaper, for example, is discarded after it has become soiled by the wearer. Optionally, a disposable diaper may include a single-use, absorbent insert, and a limited-use outer cover which may be reused several times.

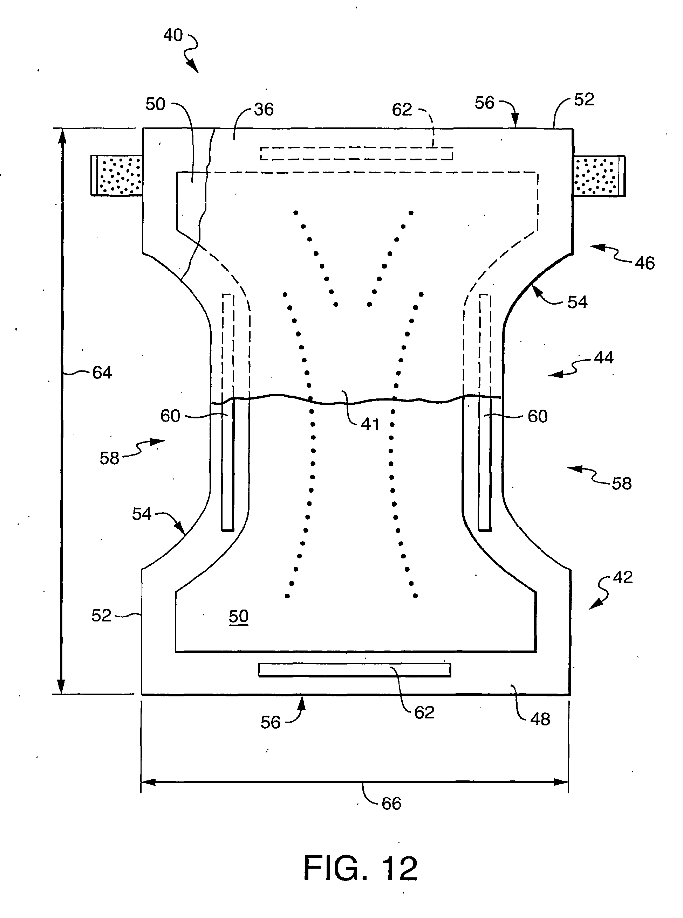

FIG. 12 illustrates a disposable diaper (40) as having a front portion (42), a rear portion (46), and a crotch portion (44) located between the front and rear portions. The disposable diaper includes an outer cover (48), a bodyside liner (41), and an absorbent structure (50) situated between the outer cover (48) and the liner (41). The outer edges of the diaper (40) define a periphery (52) with laterally opposed, longitudinally extending side edges (54); longitudinally opposed, laterally extending end edges (56); and a system of elastomeric gathering members, such as a system including leg elastics (60) and waist elastics (62). The longitudinal side edges (54) define leg openings (58) for the diaper (40), and optionally, are curvilinear and contoured. The lateral end edges (56) are illustrated as straight, but optionally, may be curvilinear. The diaper (40) may also include additional components to assist in the acquisition, distribution and storage of bodily exudates. For example, the diaper (40) may include a transport layer, such as described in U.S. Patent No. 4,798,603, issued to Meyer et al, or a surge management layer, such as described in European Patent Office Publication No. 05397703, published May 5, 1993.

With regard to the designated surfaces of the absorbent article and its components, the various upper or bodyside surfaces are configured to face toward the body of the wearer when the absorbent article is worn by the wearer for ordinary use. The various opposing or lower surfaces are configured to face away from the wearer's body when the absorbent article is worn by the wearer.

The diaper (40) generally defines a longitudinally extending length dimension (64), and a laterally extending width dimension (66), as representatively illustrated in FIG. 12. The diaper may have any desired shape, such as rectangular, I-shaped, a generally hourglass shape, or a T-shape.

The outer cover (48) and the liner (41) may be generally coextensive (e.g., FIG. 12), or optionally, may be non-coextensive. Either or both of the outer cover (48) and the liner (41) may have length and width dimensions which are generally larger than those of the absorbent structure (50) and extend beyond the corresponding dimensions of the absorbent structure (50) to provide longitudinal side edges (54) and lateral end edges (56) which may be connected or otherwise associated together in an operable manner. As used herein when describing the liner (41) in relation to the outer cover (48) and vice versa, the term "associated encompasses configurations in which the liner (41) is directly joined to the outer cover (48), and configurations where the liner (41) is indirectly joined to the outer cover (48) by affixing portions of the liner (41) to intermediate members which in turn are affixed to at least portions of the outer cover (48). The liner (41) and the outer cover (48) can, for example, be joined to each other in at least a portion of the diaper periphery (52) by attachment mechanisms (not shown) such as adhesive bonds, sonic bonds, thermal bonds, pinning, stitching, or a variety of other attachment techniques known in the art, as well as combinations thereof.

Various woven and nonwoven fabrics may be used for the liner (41). For example, the liner (41) may be composed of a meltblown or spunbonded web of polyolefin fibers. The liner (41) may also be a bonded-carded web composed of natural and/or synthetic fibers. The liner (41) may be composed of a substantially hydrophobic material, and the hydrophobic material may, optionally, be treated with a surfactant, or otherwise processed, to impart a desired level of wettability and hydrophilicity. Specifically, the liner (41) may be a nonwoven, spunbond, polypropylene fabric composed of about 2.8 to about 3.2 denier fibers formed into a web having a basis weight of about 22 gsm and a density of about 0.06 g/cc.

The liner (41) may also be surface treated with about 0.3 weight percent of a surfactant mixture that contains a mixture of AHCOVEL Base N-62 surfactant and GLUCOPON 220UP surfactant in about a 3:1 ratio based on a total weight of the surfactant mixture. The AHCOVEL Base N-62 surfactant is purchased from Hodgson Textile Chemicals Inc., a business having offices in Mount Holly, North Carolina, and includes a blend of hydrogenated ethoxylated castor oil and sorbitan monooleate in a 55:45 weight ratio. The GLUCOPON 220UP surfactant is purchased from Henkel Corporation, Gulph Mills, Pennsylvania, and includes alkyl polyglycoside. The surfactant may also include additional ingredients such as aloe. The surfactant may be applied by any conventional means, such as spraying, printing, brush coating, foam or the like. The surfactant may be applied to the entire liner (41) or may be selectively applied to particular sections of the liner (41), such as the medial section along the longitudinal centerline of a diaper, to provide greater wettability of such sections.

The outer cover (48) may suitably be composed of a material which is either liquid permeable or liquid impermeable. It is generally desirable that the outer cover (48) be formed from a material which is substantially liquid impermeable. For example, a typical outer cover (48) can be manufactured from a thin plastic film or other flexible liquid impermeable material. For example, the outer cover (48) may be formed from a polyethylene film having a thickness of from about 0.012 mm (0.5 mil) to about 0.051 mm (2.0 mils). If desirous of presenting the outer cover (48) with a more cloth-like feel, the outer cover (48) may include a polyethylene film having laminated to the lower or outer surface thereof a nonwoven web, such as a spunbond web of polyolefin fibers. For example, a polyethylene film having a thickness of about 0.015 mm (0.6 mil) may have thermally laminated thereto a spunbond web of polyolefin fibers, which fibers have a thickness of about 1.5 to about 2.5 denier (167 to 278 x 10⁻⁹ kg m⁻¹) per filament, which nonwoven web has a basis weight of about 24 gsm (0.7 osy). Methods of forming such cloth-like outer covers are known to those skilled in the art.,

Further, the outer cover (48) may be formed of a woven or nonwoven fibrous web layer which has been totally or partially constructed or treated to impart a desired level of liquid impermeability to selected regions that are adjacent or proximate the absorbent structure (50). Still further, the outer cover (48) may optionally be composed of micro-porous "breathable" material which permits vapors to escape from the absorbent structure (50) while still preventing liquid exudates from passing through the outer cover (48).

The absorbent structure (50) may have any of a number of shapes. For example, the absorbent structure (50) may be rectangular, I-shaped or T-shaped. It is generally desired that the absorbent structure (50) be narrower in the crotch portion than the rear or front portion(s).

It will be appreciated that details of the absorbent structure (50) of the invention, given for purposes of illustration, are not to be construed as limiting the scope of this invention. Although only a few exemplary aspects of this invention have been described in detail above, those skilled in the art will readily appreciate that many modifications are possible in the exemplary aspects without materially departing from the novel teachings and advantages of this invention. Accordingly, all such modifications are intended to be included within the scope of this invention, which is defined in the following claims and all equivalents thereto. Further, it is recognized that many aspects may be conceived that do not achieve all of the advantages of some aspects, particularly of the preferred aspects, yet the absence of a particular advantage should not be construed to necessarily mean that such an aspect is outside the scope of the present invention.

## Claims

1. An absorbent structure (50) comprising:
opposing first and second layers (20, 22), the first layer (20) comprising thermoplastic material, the second layer (22) comprising thermoplastic material; and
an absorbent core (24) located between the first and second layers (20, 22), said absorbent core comprising a fibrous matrix of cellulosic woodpulp fibres (27) combined with particles of a superabsorbent material (28);
the first layer (20) and the second layer (22) of the absorbent structure having thermoplastic point bonds (26) therein, wherein at least a portion of the thermoplastic point bonds (26) are liquid stable, and at least a portion of the thermoplastic point bonds (26) operatively join the first and second layers (20, 22) through the absorbent core (24);
wherein the first layer (20) is a liner (41).

2. A disposable absorbent article comprising the absorbent structure of claim 1 and an outer cover (48).

3. The absorbent article of claim 2 wherein the liner (41) is superposed over at least a portion of the outer cover (48).

4. The absorbent structure or absorbent article of claim 1, 2 or 3 further comprising a web (30) of thermoplastic material located between the first and second layers (20, 22).

5. The absorbent structure or absorbent article of claim 4, wherein the web (30) of thermoplastic material is adjacent the first layer (20).

6. The absorbent structure or absorbent article of any preceding claim,
wherein the first layer (20) is a portion of a web and the second layer (22) is a second portion of the web.

7. The absorbent structure or absorbent article of any preceding claim,
wherein the absorbent core (24) contains no less than about 40 weight percent of superabsorbent material.

8. The absorbent structure or absorbent article of any of claims 1 to 6, wherein the absorbent core (24) contains no less than about 60 weight percent of superabsorbent material.

9. The absorbent structure or absorbent article of any of claims 1 to 6, wherein the absorbent core (24) contains no less than about 80 weight percent of superabsorbent material.

10. The absorbent structure or absorbent article of any preceding claim,
wherein the thermoplastic point bonds (26) are ultrasonic bonds.

11. The absorbent structure or absorbent article of any preceding claim,
wherein the thermoplastic point bonds (26) remain intact when the absorbent structure (50) is saturated.

12. The absorbent structure or absorbent article of any preceding claim,
wherein the thermoplastic point bonds (26) operatively join materials having a combined thermoplastic basis weight of at least 50 gsm.

13. The absorbent structure or absorbent article of any preceding claim,
wherein the first layer (20) is stretchable.

14. The absorbent structure or absorbent article of claim 13, wherein the first layer (20) provides a stretch elongation that is no less than about 20%.

15. The absorbent structure or absorbent article of any preceding claim,
wherein at least a portion of the absorbent structure (70) has a basis weight no greater than about 700 gsm, preferably no greater than about 600 gsm.

16. The absorbent structure or absorbent article of any preceding claim, wherein the thermoplastic point bonds (26) are no less than about 2 mm apart.

17. The absorbent structure or absorbent article of any preceding claim, wherein the thermoplastic point bonds (26) are no less than about 3 mm apart and the thermoplastic point bonds (26) are no more than about 20 mm apart.

18. The absorbent structure or absorbent article of any preceding claim, wherein the thermoplastic point bonds (26) are no greater than about 3 mm in diameter.

19. The absorbent structure or absorbent article of any of claims 1 to 17, wherein the thermoplastic point bonds (26) are no less than about 1 mm in diameter and the thermoplastic point bonds (26) are no greater than about 4 mm in diameter.

## Patentansprüche

1. Absorbierende Struktur (50), enthaltend:
einander gegenüberliegend eine erste und eine zweite Lage (20, 22), welche erste Lage (20) thermoplastisches Material umfasst, welche zweite Lage (22) thermoplastisches Material umfasst; und
einen absorbierenden Kern (24), der zwischen der ersten und der zweiten Lage (20, 22) angeordnet Ist, welcher absorbierende Kern eine Fasermatrix aus Zellulose-Holzstofffasern (27), kombiniert mit Teilchen eines superabsorbierenden Materials (28) enthält;
wobei die erste Lage (20) und die zweite Lage (22) der absorbierenden Struktur thermoplastische Punktverbindungen (26) aufweisen, wobei mindestens ein Teil der thermoplastischen Punktverbindungen (26) flüssigkeitsstabil ist und mindestens ein Teil der thermoplastischen Punktverbindungen (26) die erste und die zweite Lage (20, 22) durch den absorbierenden Kern (20) miteinander operativ verbindet;
wobei die erste Lage (20) eine Auskleidung (41) ist.

2. Absorbierender Wegwerfartikel, enthaltend die absorbierende Struktur nach Anspruch 1 und eine äußere Abdeckung (48).

3. Absorbierender Artikel nach Anspruch 2, wobei die Auskleidung (41) über mindestens einen Teil der äußeren Abdeckung (48) gelegt ist.

4. Absorbierende Struktur oder absorbierender Artikel nach Anspruch 1, 2 oder 3, ferner enthaltend eine Bahn (30) aus thermoplastischem Material, die zwischen der ersten und der zweiten Lage (20, 22) angeordnet ist.

5. Absorbierende Struktur oder absorbierender Artikel nach Anspruch 4, wobei die Bahn (30) aus thermoplastischem Material der ersten Lage (20) benachbart ist.

6. Absorbierende Struktur oder absorbierender Artikel nach einem der vorhergehenden Ansprüche, wobei die erste Lage (20) ein Teil einer Bahn ist und die zweite Lage (22) ein zweiter Teil der Bahn ist.

7. Absorbierende Struktur oder absorbierender Artikel nach einem der vorhergehenden Ansprüche, wobei der absorbierende Kern (24) nicht weniger als etwa 40 Gew.-% superabsorbierendes Material enthält.

8. Absorbierende Struktur oder absorbierender Artikel nach einem der Ansprüche 1 bis 6, wobei der absorbierende Kern (24) nicht weniger als etwa 60 Gew.-% superabsorbierendes Material enthält.

9. Absorbierende Struktur oder absorbierender Artikel nach einem der Ansprüche 1 bis 6, wobei der absorbierende Kern (24) nicht weniger als etwa 80 Gew.-% superabsorbierendes Material enthält.

10. Absorbierende Struktur oder absorbierender Artikel nach einem der vorhergehenden Ansprüche, wobei die thermoplastischen Punktverbindungen (26) Ultraschallverbindungen sind.

11. Absorbierende Struktur oder absorbierender Artikel nach einem der vorhergehenden Ansprüche, wobei die thermoplastischen Punktverbindungen (26) intakt bleiben, wenn die absorbierende Struktur (50) gesättigt ist.

12. Absorbierende Struktur oder absorbierender Artikel nach einem der vorhergehenden Ansprüche, wobei die thermoplastischen Punktverbindungen (26) Materialien operativ verbinden, die ein kombiniertes thermoplastisches Basisgewicht von mindestens 50 g/m² haben.

13. Absorbierende Struktur oder absorbierender Artikel nach einem der vorhergehenden Ansprüche, wobei die erste Lage (20) dehnbar ist.

14. Absorbierende Struktur oder absorbierender Artikel nach Anspruch 13, wobei die erste Lage (20) eine elastische Dehnung bietet, die nicht geringer als etwa 20 % ist.

15. Absorbierende Struktur oder absorbierender Artikel nach einem der vorhergehenden Ansprüche, wobei mindestens ein Teil der absorbierenden Struktur (70) ein Basisgewicht hat, das nicht größer als etwa 700 g/m² ist, vorzugsweise nicht größer als etwa 600 g/m².

16. Absorbierende Struktur oder absorbierender Artikel nach einem der vorhergehenden Ansprüche, wobei die thermoplastischen Punktverbindungen (26) nicht weniger als etwa 2 mm beabstandet sind.

17. Absorbierende Struktur oder absorbierender Artikel nach einem der vorhergehenden Ansprüche, wobei die thermoplastischen Punktverbindungen (26) nicht weniger als etwa 3 mm beabstandet sind und die thermoplastischen Punktverbindungen (26) nicht mehr als etwa 20 mm beabstandet sind.

18. Absorbierende Struktur oder absorbierender Artikel nach einem der vorhergehenden Ansprüche, wobei der Durchmesser der thermoplastischen Punktverbindungen (26) nicht größer als etwa 3 mm ist.

19. Absorbierende Struktur oder absorbierender Artikel nach einem der Ansprüche 1 bis 17, wobei der Durchmesser der thermoplastischen Punktverbindungen (26) nicht kleiner als etwa 1 mm ist und der Durchmesser der thermoplastischen Punktverbindungen (26) nicht größer als etwa 4 mm ist.

## Revendications

1. Structure absorbante (50) comprenant :
des première et seconde couches opposées (20, 22), la première couche (20) comprenant une matière thermoplastique, la seconde couche (22) comprenant une matière thermoplastique ; et
un coeur absorbant (24) situé entre les première et seconde couches (20, 22), ledit coeur absorbant comprenant une matrice fibreuse de fibres de pâte cellulosique de bois (27) combinées à des particules d'une matière superabsorbante (28) ;
la première couche (20) et la seconde couche (22) de la structure absorbante comportant des liaisons ponctuelles thermoplastiques (26), dans laquelle au moins une partie des liaisons ponctuelles thermoplastiques (26) sont stables en présence de liquide, et au moins une partie des liaisons ponctuelles thermoplastiques (26) joignant opérationnellement les première et seconde couches (20, 22) par le biais du coeur absorbant (24) ;
dans laquelle la première couche (20) est une doublure (41).

2. Article absorbant jetable comprenant la structure absorbante selon la revendication 1 et un revêtement externe (48).

3. Article absorbant selon la revendication 2, dans lequel la doublure (41) est superposée par-dessus au moins une partie du revêtement externe (48).

4. Structure absorbante ou article absorbant selon la revendication 1, 2 ou 3, comprenant en outre une bande (30) de matière thermoplastique située entre les première et seconde couches (20, 22).

5. Structure absorbante ou article absorbant selon la revendication 4, dans laquelle ou dans lequel la bande (30) de matière thermoplastique est adjacente à la première couche (20).

6. Structure absorbante ou article absorbant selon l'une quelconque des revendications précédentes, dans laquelle ou dans lequel la première couche (20) est une partie d'une bande et la seconde couche (22) est une seconde partie de la bande.

7. Structure absorbante ou article absorbant selon l'une quelconque des revendications précédentes, dans laquelle ou dans lequel le coeur absorbant (24) ne contient pas moins d'environ 40 pour cent en poids de matière superabsorbante.

8. Structure absorbante ou article absorbant selon l'une quelconque des revendications 1 à 6, dans laquelle ou dans lequel le coeur absorbant (24) ne contient pas moins d'environ 60 pour cent en poids de matière superabsorbante.

9. Structure absorbante ou article absorbant selon l'une quelconque des revendications 1 à 6, dans laquelle ou dans lequel le coeur absorbant (24) ne contient pas moins d'environ 80 pour cent en poids de matière superabsorbante.

10. Structure absorbante ou article absorbant selon l'une quelconque des revendications précédentes, dans laquelle ou dans lequel les liaisons ponctuelles thermoplastiques (26) sont des liaisons ultrasoniques.

11. Structure absorbante ou article absorbant selon l'une quelconque des revendications précédentes, dans laquelle ou dans lequel les liaisons ponctuelles thermoplastiques (26) restent intactes quand la structure absorbante (50) est saturée.

12. Structure absorbante ou article absorbant selon l'une quelconque des revendications précédentes, dans laquelle ou dans lequel les liaisons ponctuelles thermoplastiques (26) joignent opérationnellement les matières ayant un poids de base de thermoplastiques combiné d'au moins 50 gsm.

13. Structure absorbante ou article absorbant selon l'une quelconque des revendications précédentes, dans laquelle ou dans lequel la première couche (20) est étirable.

14. Structure absorbante ou article absorbant selon la revendication 13, dans laquelle ou dans lequel la première couche (20) fournit un allongement à l'étirage non inférieur à environ 20%.

15. Structure absorbante ou article absorbant selon l'une quelconque des revendications précédentes, dans laquelle ou dans lequel au moins une partie de la structure absorbante (70) a un poids de base non supérieur à environ 700 gsm, mieux encore non supérieur à environ 600 gsm.

16. Structure absorbante ou article absorbant selon l'une quelconque des revendications précédentes, dans laquelle ou dans lequel les liaisons ponctuelles thermoplastiques (26) sont espacées par au moins environ 2 mm.

17. Structure absorbante ou article absorbant selon l'une quelconque des revendications précédentes, dans laquelle ou dans lequel les liaisons ponctuelles thermoplastiques (26) sont espacées par au moins environ 3 mm et les liaisons ponctuelles thermoplastiques (26) sont espacées par au plus environ 20 mm.

18. Structure absorbante ou articule absorbant selon l'une quelconque des revendications précédentes, dans laquelle ou dans lequel le diamètre des liaisons ponctuelles thermoplastiques (26) est au plus d'environ 3 mm.

19. Structure absorbante ou article absorbant selon l'une quelconque des revendications 1 à 17, dans laquelle ou dans lequel le diamètre des liaisons ponctuelles thermoplastiques (26) est au moins d'environ 1 mm et le diamètre des liaisons ponctuelles thermoplastiques (26) est au plus d'environ 4 mm.
